Europäisches Patentamt

European Patent Office

Office européen des brevets

(11) Veröffentlichungsnummer: **0 023 071**
**B1**

(12) **EUROPÄISCHE PATENTSCHRIFT**

(45) Veröffentlichungstag der Patentschrift:
06.04.83

(51) Int. Cl.³: **C 07 C 29/04, B 01 J 21/16, B 01 J 27/16, B 01 J 23/24**

(21) Anmeldenummer: **80200715.3**

(22) Anmeldetag: **22.07.80**

(54) Verfahren zur Herstellung eines Katalysators aus Tonmaterialien für die Hydratation von Olefinen und nach diesem Verfahren hergestellter Katalysator.

(30) Priorität: **24.07.79 DE 2929919**

(43) Veröffentlichungstag der Anmeldung:
**28.01.81 Patentblatt 81/4**

(45) Bekanntmachung des Hinweises auf die Patenterteilung:
**06.04.83 Patentblatt 83/14**

(84) Benannte Vertragsstaaten:
**BE DE FR GB IT NL**

(56) Entgegenhaltungen:
**DE-B-1 156 772**
**US-A-3 076 036**
**US-A-3 554 926**
**US-A-3 862 249**

(73) Patentinhaber: **CHEMISCHE WERKE HÜLS AG, Postfach 1320, D-4370 Marl 1 (DE)**

(72) Erfinder: **Sommer, August, Dr., Birnenbruchstrasse 10, D-4690 Herne 1 (DE)**
Erfinder: **Heitmann, Wilhelm, Dr., Bahnhofstrasse 7c, D-4690 Herne 1 (DE)**
Erfinder: **Brücker, Rainer, Dr., Unterspredey 65, D-4620 Castrop-Rauxel (DE)**

(74) Vertreter: **Steil, Hanna, Dipl.-Chem. et al, RSP PATENTE - PB 15 Postfach 1320, D-4370 Marl 1 (DE)**

## Verfahren zur Herstellung eines Katalysators aus Tonmineralien für die Hydratation von Olefinen und nach diesem Verfahren hergestellter Katalysator

Beschreibung und Beispiele

Es ist bekannt, dass sich Olefine in der Gasphase bei erhöhten Drücken mit Wasserdampf zu Alkoholen umsetzen lassen. Besondere technische Bedeutung haben solche Verfahren bei der Herstellung von Äthylalkohol aus Äthylen und Isopropylalkohol aus Propylen erlangt. Die Synthese dieser Alkohole wird in Gegenwart von Katalysatoren durchgeführt, und zwar dient in aller Regel als Katalysator Phosphorsäure, die auf Trägern aufgebracht ist.

Bekannt sind Trägermaterialien entweder auf der Basis reiner Kieselsäure (z.B. Kieselgur oder Kieselgel) oder auf der Basis von Kieselsäure mit mehr oder weniger grossem Tonerdegehalt, wie z.B. calcinierte Diatomeenerde, deren Struktur durch Ton oder tonartige Stoffe zusammengehalten wird.

Bei den Trägern auf Basis reiner Kieselsäure ist die Festigkeit über längere Standzeiten problematisch. Die tonerdehaltigen Materialien zeichnen sich zwar durch eine bessere mechanische Festigkeit aus, sie haben jedoch bei zu hohem Tonerdegehalt den Nachteil, dass während der Reaktion das Aluminiumoxid durch die Einwirkung der Phosphorsäure herausgelöst wird.

In DE-PS 1156772 wurde nun ein Verfahren beschrieben, einen tonerdehaltigen Träger für die bei der Olefinhydratation als Katalysator verwendete Phosphorsäure herzustellen, in dem geformte Kontaktkörper aus mineralischen Tonerdesilikaten derart mit Mineralsäure behandelt werden, dass der Aluminiumoxidgehalt vorzugsweise auf zwischen 1 und 5 Gew.% abgesenkt wird. Dieses Material weist im allgemeinen sowohl die erforderliche mechanische Festigkeit auf als auch einen ausreichend niedrigen Rest-Aluminiumoxidgehalt zur Vermeidung des Herauslösens. Andererseits wurde beim Einsatz von handelsüblichen Kontaktkörpern für die Herstellung von Katalysatorträgern für die Hydratation von Olefinen beobachtet, dass ohne Vorauswahl des Rohstoffs stark unterschiedliche Katalysator-Aktivitäten gefunden werden.

Schliesslich war es gelungen, auch auf der Basis grobporiger Kieselgele Träger für Phosphorsäure mit hoher Hydratationsaktivität und ausreichender mechanischer Festigkeit zu entwickeln, z.B. DE-OS 2625705 und DE-OS 2719055. Allerdings verbleibt als Nachteil dieser Träger auf der Basis amorpher Kieselsäure, dass bei längerem Aussetzen gegen die Bedingungen der Hydratationsreaktion die amorphe Kieselsäure teilweise zu Cristobalit und Quarz kristallisiert, was mit starker Verminderung der spezifischen Oberfläche und damit der katalytischen Aktivität, und zwar irreversibel, verbunden ist, sowie mit einer Abnahme der mechanischen Festigkeit.

Ein weiterer Nachteil aller bisher eingesetzten Hydratationskatalysatoren auf Basis Phosphorsäure auf silikatischem Träger ist das langsame Abnehmen der Aktivität durch ausgetragene Phosphorsäure, die im kontinuierlichen Betrieb kontinuierlich mit Lauge neutralisiert werden muss, um Korrosionswirkungen des sauren Rohalkohols auf nachgeschaltete Destillationsapparate zu vermeiden.

Durch die inzwischen entwickelte kontinuierliche Einspritzung der ausgetragenen Phosphorsäuremenge gemäss DE-OS 2658946 konnte zwar der laufende Aktivitätsverlust weitgehend vermieden und damit die Katalysator-Lebensdauer beträchtlich verlängert werden. Damit sind aber entsprechende Anforderungen an die Lebensdauer des Trägers zu stellen, so dass die Verwendung solcher Träger ausscheidet, bei denen unter Reaktionsbedingungen Kristallisationen unter Verminderung der katalytischen Aktivität auf irreversible Weise ablaufen und die mechanische Festigkeit im Laufe der Zeit abnimmt.

Wie Patentanmeldung DE-OS 2908491 zeigt, lässt sich aus Tonmineralien dann ein Träger für einen Hydratationskatalysator gleichbleibend hoher katalytischer Aktivität erhalten, wenn durch sorgfältige Auswahl des Rohstoffs Vorsorge dafür getroffen wird, dass das Material in hohem Masse aus Montmorillonit besteht, was dazu führt, dass nach Formung, Auslaugung und Tränkung die aktive Oberfläche, an der also die Hydratation des Olefins stattfinden kann, gross ist.

Die Patentanmeldung DE-OS 2908491 betrifft ein Verfahren zur Herstellung eines Katalysators aus Tonmineralien für die Hydratation von Olefinen mit 2-3 C-Atomen zu den entsprechenden Alkoholen, aus Phosphorsäure und Trägermaterial — sowie den danach hergestellten Katalysator — bei dem man einen im wesentlichen montmorillonithaltigen Ton, der mit nicht mehr als 3% Begleitmineralien, wie Quarz, Feldspat und Glimmer, verunreinigt ist und bis 0,5% $K_2O$ enthalten kann, zunächst in einer ersten Stufe mit Säure behandelt, bis er einen $Al_2O_3$-Gehalt von 13-18 Gew.% aufweist und gegebenenfalls durch Zugabe von gefällter Tonerde den $Al_2O_3$-Gehalt auf 16-18 Gew.% einstellt, wobei sich eine Oberfläche von 200-400 m²/g, bevorzugt 240-300 m²/g, ergibt, dann bei einem Gesamtwassergehalt von 20-35% durch Pressen formt, bei 500-800° C calciniert und anschliessend das geformte Trägermaterial in einer zweiten Stufe mit Säure bis auf einen $Al_2O_3$-Gehalt von 1-5 Gew.%, bevorzugt 1-3 Gew.%, behandelt, wobei sich eine Oberfläche von 150-250 m²/g, bevorzugt 180-220 m²/g, ergibt und den so erhaltenen Träger in bekannter Weise mit Phosphorsäure tränkt.

Anstelle von Montmorillonit kann dabei auch ein anderes Mineral der Montmorillonitgruppe eingesetzt werden, das kein Kalium, aber das Montmorillonit-Kristallgitter enthält.

Es ist dabei auch möglich, anstelle eines noch nicht säurebehandelten montmorillonithaltigen Tons eine bereits einmal säurebehandelte Bleicherde, hergestellt aus stark montmorillonithaltigem Ton, einzusetzen, wobei sich dann die erste Säurebehandlung erübrigt. Diese Bleicherde soll weniger als 0,1% $K_2O$ enthalten, das Gewichtsverhältnis $(Al_2O_3+Fe_2O_3):SiO_2$ soll 1:3,5 bis 1:4,5 betragen. Falls nötig, kann der $Al_2O_3$-Gehalt der Bleicherde durch Zugabe von gefällter Tonerde auf die erforderlichen 16-18 Gew.% eingestellt werden.

Die so gezielt hergestellten Katalysatoren bzw. Katalysatorträger aus montmorillonithaltigem Ton zeigen gegenüber solchen, hergestellt aus geformten Kontaktkörpern auf Basis mineralischer Tonerdesilikate unterschiedlicher Provenienz, eine erhöhte Aktivität, d.h. es werden pro Stunde und Liter Katalysatorschüttung etwa 105-110 g Äthanol bzw. ca. 300 g Isopropylalkohol gewonnen. Diese erhöhte Aktivität kann jedoch nur über einen längeren Zeitraum aufrechterhalten werden, wenn die ausgetragene Phosphorsäure, die bei Äthanol etwa 0,07 g pro h und l Katalysatorschüttung beträgt, bei Isopropylalkohol etwa 0,01 g pro h und l Katalysatorschüttung, durch kontinuierliche Zugabe der gleichen Säuremenge ausgeglichen wird. Diese ausgetragene Säure muss darüber hinaus noch mit Lauge neutralisiert werden.

Die mechanische Festigkeit der Katalysatoren liegt in der Grössenordnung von 7-9 kg/Kugel, was für das Beschicken von üblichen Reaktoren ausreichend ist.

Überraschenderweise wurde nun gefunden, dass sich durch Zugabe von 5-15 Gew.% auf die Gesamt-Trockensubstanz, bezogen von einem Oxid oder einer Mischung mehrerer Oxide von Elementen der VI. Nebengruppe des Periodensystems weitere Eigenschaften des fertigen Katalysators verbessern lassen.

Gegenstand der Erfindung ist daher ein Verfahren zur Herstellung eines Katalysators aus Tonmineralien für die Hydratation von Olefinen mit 2-3 C-Atomen zu den entsprechenden Alkoholen aus Phosphorsäure und Trägermaterial, dadurch gekennzeichnet, dass man einen im wesentlichen montmorillonithaltigen Ton, der mit nicht mehr als 3% Begleitmineralien, wie Quarz, Feldspat und Glimmer, verunreinigt ist und bis 0,5% $K_2O$ enthalten kann, zunächst in einer ersten Stufe mit Säure behandelt, bis er, gegebenenfalls durch Zugabe von gefällter Tonerde, einen $Al_2O_3$-Gehalt von 13-18 Gew.% und eine Oberfläche von 200-400 m²/g, bevorzugt 240-300 m²/g; aufweist, danach 5-15 Gew.%, bezogen auf die Gesamttrockensubstanz, an einem oder mehreren Oxiden von Metallen der VI. Nebengruppe des Periodensystems zugibt, dann bei einem Gesamtwassergehalt von 20-35% durch Pressen formt, bei 500-800° C calciniert und anschliessend das geformte Trägermaterial in einer zweiten Stufe mit Säure behandelt, bis es einen $Al_2O_3$-Gehalt von 1-5 Gew.%, bevorzugt 1-3 Gew.%, und eine Oberfläche von 150-250 m²/g, bevorzugt 180-220 m²/g, aufweist, und den so erhaltenen Träger in bekannter Weise mit Phosphorsäure tränkt.

Es können auch andere Minerale der Montmorillonitgruppe, die kein Kalium, aber das Montmorillonit-Kristallgitter enthalten, eingesetzt werden.

Man kann auch anstelle eines noch nicht säurebehandelten montmorillonithaltigen Tons eine bereits einmal säurebehandelte Bleicherde, hergestellt aus stark montmorillonithaltigem Ton, einsetzen, wobei sich dann die erste Säurebehandlung erübrigt. Diese Bleicherde soll weniger als 0,1% $K_2O$ enthalten, das Gewichtsverhältnis $(Al_2O_3+Fe_2O_3):SiO_2$ soll 1:3,5 bis 1:4,5 betragen. Falls nötig, kann der $Al_2O_3$-Gehalt der Bleicherde durch Zugabe von gefällter Tonerde auf die erforderlichen 16-18 Gew.% eingestellt werden.

Nach der Erfindung werden folgende Vorteile erreicht:

1. Die mechanische Festigkeit des Katalysators steigt auf etwa 11-13 kg/Kugel. Dies hat Bedeutung bei Einsatz höherer Katalysatorschüttungen.

2. Die Katalysatoraktivität steigt auf etwa 130 g Äthanol und etwa 350 g Isopropylalkohol pro h und l Katalysatorschüttung.

3. Die Phosphorsäureaustragung geht auf etwa die Hälfte zurück, also beim Äthanol auf etwa 0,035 g pro h und l Katalysatorschüttung, beim Isopropylalkohol auf etwa 0,005 g pro h und l Katalysatorschüttung.

Neben der festeren Bindung der Phosphorsäure an den Träger über die Heteropolysäure des Metalles der VI. Nebengruppe des Periodensystems ist vor allem die Tatsache überraschend, dass sich die Oxide der Metalle der VI. Nebengruppe des Periodensystems in das Silikatgitter in einer solchen Weise einbauen lassen, dass sie bei der anschliessenden Säurebehandlung überhaupt nicht herausgelöst werden. So erfolgt offensichtlich auch schon eine Heteropolysäurebildung zwischen dem Silicium und den Oxiden der Elemente der VI. Nebengruppe beim Calcinieren unter den angegebenen Bedingungen.

*Beispiel 1:*

Ein gemahlener natürlicher Rohton, der aufgrund einer Laboruntersuchung so ausgewählt wurde, dass bei einer einstündigen Behandlung mit 20%iger Salzsäure bei 82° C nicht mehr als 5 g $K_2O$ pro kg eingesetzte Trockensubstanz extrahiert werden, wurde eine Stunde lang mit 20%iger Salzsäure auf 82° C erhitzt, wobei die Säuremenge so bemessen wurde, dass auf 1 kg Ton 8,4 Mol HCl kamen, säurefrei gewaschen und getrocknet. Dabei wurde ein Material mit einem Rest-Aluminiumoxidgehalt von 16 Gew.% und einer spezifischen Oberfläche von 300 m²/g erhalten:

Zu 100 Teilen dieses getrockneten Materials wurden 15 Teile Wolframoxid ($WO_3$) gegeben, so dass die Mischung 13 Gew.% Wolframoxid enthielt. Nach Anfeuchten mit 25% Wasser auf die Gesamtmenge bezogen (das ist Zugabe von 33% der Trockensubstanz als Wasser) wurde das Material in zylindrische Formen von 4 mm Durchmesser und 4 mm Höhe gepresst und verfestigt durch 3 Stunden langes Erhitzen auf 600° C.

Die so erhaltenen geformten Kontaktkörper wurden insgesamt zweimal für eine Stunde mit 20%iger Salzsäure bei 100-110° C behandelt und mit Wasser säurefrei gewaschen. Nach dem Trocknen bei etwa 110-120° C wurde in den Zylindern ein Aluminiumoxidgehalt von 2,7 Gew.% gefunden, die spezifische Oberfläche betrug 215 m²/g. Der Gehalt an Wolframoxid hatte sich auf 15 Gew.% erhöht.

Diese Formkörper wurden sodann mit 60 gew.%iger Phosphorsäure überflutet, die zwei Stunden einwirkte, danach wurde erneut bei etwa 110-120° C getrocknet. Die so behandelten Zylinder hatten einen $H_3PO_4$-Gehalt von 35 Gew.%. Die mittlere Druckfestigkeit betrug 11 kg/Zylinder.

Beim Einsatz dieses so fertiggestellten Hydratationskatalysators zur Synthese von Äthanol aus Äthylen und Wasser in der Gasphase konnte eine Katalysatorausbeute von 130 g Äthanol pro h und l Katalysatorschüttung erzielt werden. Die Austragung an Phosphorsäure unter Reaktionsbedingungen betrug 0,035 g pro h und l Katalysatorschüttung.

*Beispiel 2:*

Eine hochaktive Bleicherde mit einer spezifischen Oberfläche von 350 m²/g und folgender chemischer Analyse — 72,5% $SiO_2$, 14,0% $Al_2O_3$, 4,0% $Fe_2O_3$, 1,5% MgO, 0,8% CaO, 7,2% Glühverlust, $K_2O$ 0,1% — wurde auf 100 Teile mit 8 Teilen Chromoxid ($CrO_3$) gemischt, so dass die Mischung 7,4% Chromoxid enthielt. Nach Anfeuchten mit 30% Wasser auf die Gesamtmenge bezogen (das ist Zugabe von 43% der Trockensubstanz als Wasser) wurde zu Kugeln von 4 mm Durchmesser verpresst und durch 3 Stunden langes Erhitzen auf 600° C verfestigt.

Die so erhaltenen geformten Kontaktkörper wurden insgesamt zweimal für eine Stunde mit 20%iger Salzsäure bei 100-110° C behandelt und mit Wasser säurefrei gewaschen. Nach dem Trocknen bei etwa 110-120° C wurde in den Kugeln ein Aluminiumoxidgehalt von 1,4 Gew.% gefunden, die spezifische Oberfläche betrug 230 m²/g. Der Gehalt an Chromoxid hatte sich auf 9 Gew.% erhöht.

Diese Formkörper wurden sodann mit 60 gew.%iger Phosphorsäure überflutet, die zwei Stunden einwirkte, danach wurde erneut bei etwa 110-120° C getrocknet. Die so behandelten Kugeln hatten einen $H_3PO_4$-Gehalt von 36 Gew.%. Die mittlere Druckfestigkeit betrug 13 kg/Kugel.

Beim Einsatz dieses so fertiggestellten Hydratationskatalysators zur Synthese von Äthanol aus Äthylen und Wasser in der Gasphase konnte eine Katalysatorausbeute von 130 g Äthanol pro h und l Katalysatorschüttung erzielt werden. Die Austragung an Phosphorsäure unter Reaktionsbedingungen betrug 0,035 g pro h und l Katalysatorschüttung.

*Beispiel 3:*

Eine hochaktive Bleicherde mit einer spezifischen Oberfläche von 350 m²/g und folgender chemischer Analyse — 72,5% $SiO_2$, 14,0% $Al_2O_3$, 4,0% $Fe_2O_3$, 1,5% MgO, 0,8% CaO, 7,2% Glühverlust, $K_2O$ 0,1% — wurde auf 100 Teile mit 10 Teilen Molybdänoxid ($MoO_3$) gemischt, so dass die Mischung 9% Molybdänoxid enthielt. Nach Anfeuchten mit 30% Wasser auf die Gesamtmenge bezogen (das ist Zugabe von 43% der Trockensubstanz als Wasser) wurde zu Kugeln von 4 mm Durchmesser verpresst und durch 3 Stunden langes Erhitzen auf 600° C verfestigt.

Die so erhaltenen geformten Kontaktkörper wurden insgesamt zweimal für eine Stunde mit 20%iger Salzsäure bei 100-110° C behandelt und mit Wasser säurefrei gewaschen. Nach dem Trocknen bei etwa 110-120° C wurde in den Kugeln ein Aluminiumoxidgehalt von 1,4 Gew.% gefunden, die spezifische Oberfläche betrug 230 m²/g. Der Gehalt an Molybdänoxid hatte sich auf 11 Gew.% erhöht.

Diese Formkörper wurden sodann mit 40 gew.%iger Phosphorsäure überflutet, die zwei Stunden einwirkte, danach wurde erneut bei etwa 110-120° C getrocknet. Die so behandelten Kugeln hatten einen $H_3PO_4$-Gehalt von 27 Gew.%. Die mittlere Druckfestigkeit betrug 12 kg/Kugel.

Beim Einsatz dieses so fertiggestellten Hydratationskatalysators zur Synthese von Isopropylalkohol aus Propylen und Wasser in der Gasphase konnte eine Katalysatorausbeute von 350 g Isopropylalkohol pro h und l Katalysatorschüttung erzielt werden. Die Austragung an Phosphorsäure unter Reaktionsbedingungen betrug 0,005 g pro h und l Katalysatorschüttung.

*Beispiel 4:*

Eine hochaktive Bleicherde mit einer spezifischen Oberfläche von 350 m²/g und folgender chemischer Analyse — 75,5% $SiO_2$, 14,0% $Al_2O_3$, 4,0% $Fe_2O_3$, 1,5% MgO, 0,8% CaO, 7,2% Glühverlust, $K_2O$ 0,1% — wurde auf 10 Teile mit 3 Teilen Chromoxid ($CrO_3$), 3 Teilen Molybdänoxid ($MoO_3$), 5 Teilen Wolframoxid ($WO_3$) gemischt, so dass die Mischung insgesamt 10% an Oxiden von Elementen der VI. Nebengruppe des Periodensystems enthielt.

Nach Anfeuchten mit 30% Wasser auf die Gesamtmenge bezogen (das ist Zugabe von 43% der Trockensubstanz als Wasser) wurde zu Kugeln von 4 mm Durchmesser verpresst und durch 3 Stunden langes Erhitzen auf 600° C verfestigt.

Die so erhaltenen geformten Kontaktkörper wurden insgesamt zweimal für eine Stunde mit 20%iger Salzsäure bei 100-110° C behandelt und mit Wasser säurefrei gewaschen. Nach dem Trocknen bei etwa 110-120° C wurde in den Kugeln ein Aluminiumoxidgehalt von 1,4% gefunden, die spezifische Oberfläche betrug 230 m²/g. Der Gehalt an Oxiden von Elementen der VI. Nebengruppe des Periodensystems hatte sich auf 12 Gew.% erhöht.

Diese Formkörper wurden sodann mit 60 gew.%iger Phosphorsäure überflutet, die zwei Stunden einwirkte, danach wurde erneut bei etwa 110-120° C getrocknet. Die so behandelten Kugeln hatten einen $H_3PO_4$-Gehalt von 35 Gew.%. Die mittlere Druckfestigkeit betrug 13 kg/Kugel.

Beim Einsatz dieses so fertiggestellten Hydratationskatalysators zur Synthese von Äthanol aus Äthylen und Wasser in der Gasphase konnte eine Katalysatorausbeute von 130 g Äthanol pro h und l Katalysatorschüttung erzielt werden. Die Austragung an Phosphorsäure unter Reaktionsbedingungen betrug 0,035 g pro h und l Katalysatorschüttung.

## Patentansprüche

1. Verfahren zur Herstellung eines Katalysators aus Tonmineralien für die Hydratation von Olefinen mit 2-3 C-Atomen zu den entsprechenden Alkoholen, aus Phosphorsäure und Trägermaterial, dadurch gekennzeichnet, dass man einen Ton der im wesentlichen Montmorillonit oder andere Minerale der Montmorillonitgruppe, die kein Kalium aber das Montmorillonit-Kristallgitter enthalten und der mit nicht mehr als 3 Gew.% Begleitmineralien, wie Quarz, Feldspat und Glimmer, verunreinigt ist und bis 0,5 Gew.% $K_2O$ enthalten kann, zunächst in einer ersten Stufe mit Säure behandelt, bis er, gegebenenfalls durch Zugabe von gefällter Tonerde einen $Al_2O_3$-Gehalt von 13-18 Gew.% und eine Oberfläche von 200-400 m²/g, bevorzugt 240-300 m²/g, aufweist, danach 5-15 Gew.%, bezogen auf die Gesamttrockensubstanz, an einem oder mehreren Oxiden von Metallen der VI. Nebengruppe des Periodensystems zugibt, dann bei einem Gesamtwassergehalt von 20-35 Gew.% durch Pressen formt, bei 500-800° C calciniert und anschliessend das geformte Trägermaterial in einer zweiten Stufe mit Säure behandelt, bis es einen $Al_2O_3$-Gehalt von 1-5 Gew.%, bevorzugt 1-3 Gew.%, und eine Oberfläche von 150-250 m²/g, bevorzugt 180-220 m²/g, aufweist, und den so erhaltenen Träger in bekannter Weise mit Phosphorsäure tränkt.

2. Verfahren nach Anspruch 1, dadurch gekennzeichnet, dass von einer bereits einmal säurebehandelten hochaktiven Bleicherde, hergestellt aus einem Ton mit hohem Montmorillonitgehalt, ausgegangen wird, die weniger als 0,1 Gew.% $K_2O$ enthält, deren Gewichtsverhältnis $(Al_2O_3+Fe_2O_3)$ : $SiO_2$ 1:3,5 bis 1:4,5 beträgt, deren $Al_2O_3$-Gehalt gegebenenfalls durch Zugabe von gefällter Tonerde auf 16-18 Gew.% eingestellt wird, und deren spezifische Oberfläche 200-400 m²/g, bevorzugt 240-300· m²/g, beträgt, diese Bleicherde mit 5-15 Gew.%, bezogen auf die Gesamttrockensubstanz, an einem oder mehreren Oxiden der VI. Nebengruppe des Periodensystems vesetzt und durch Pressen in Gegenwart eines Gesamtwassergehaltes von 20-35 Gew.% geformt und nach Calcinieren zwischen 500 und 800° C einer Säurebehandlung unterzogen wird, wobei der Aluminiumoxidgehalt bis auf 1-5 Gew.%, bevorzugt 1-3 Gew.%, und die spezifische Oberfläche auf 150-250 m²/g, bevorzugt 180-220 m²/g, gesenkt wird, wonach man den so erhaltenen Kontaktträger in bekannter Weise mit Phosphorsäure tränkt.

3. Katalysator aus Tonmineralien für die Hydratation von Olefinen, hergestellt nach den Ansprüchen 1 und 2.

## Claims

1. Process for the preparation of a catalyst from clay minerals for the hydratisation of olefins with 2-3 C-atoms to the corresponding alcohols, from phosphoric acid and carrier material, characterised in that a clay containing essentially montmorillonite or other minerals of the montmorillonite group, which do not contain potassium, but the montmorillonite crystal lattice, and contaminated by no more than 3% accompanying minerals such as quartz, feldspar and mica, and containing up to 0,5% $K_2O$, is produced in a first step with acid until it shows an $Al_2O_3$-content of 13-18% by weight — if necessary, by adding precipitated clay —, and a surface area of 200-400 m²/g, preferably 240-300 m²/g, and then 5-15% by weight, based on the total dry substance, of one or several oxides of metals of periodic group VI, are added, and at a total water content of 20-35% it is pressed into a form, calcined at 500-800° C, and then the formed carrier material is treated with acid in a second step, until the $Al_2O_3$-content reaches 1-5% by weight, preferably 1-3% by weight, and has a surface area of 150-250 m²/g, preferably 180-220 m²/g, and the resulting carrier is impregnated in a known manner with phosphoric acid.

2. Process according to claim 1, characterised in that the raw material is a highly active fuller's earth, already being acid-treated once, made from a clay with a high montmorillonite content, having less than 0,1% $K_2O$, whose weight ratios $(Al_2O_3+Fe_2O_3)$ : $SiO_2$ are 1:3,5 to 1:4,5, whose $Al_2O_3$-content can be brought to 16-18%, if necessary, by adding precipitated clay, and whose specific surface area is 200-400 m²/g, preferably 240-300 m²/g; this fuller's earth with 5-15% by weight, based on the total dry substance, of one or several oxides of periodic group VI is mixed and pressed in the presence of a total water content of 20-35% into forms and after calcining at between 500 and 800° C is treated with an acid, whereby the aluminium oxide content is up to 1-5% by weight, preferably 1-3% by weight, and the specific surface area is reduced to 150-250 m²/g, preferably 180-220 m²/g, whereupon the so produced contact bodies are impregnated with phosphoric acid in a known manner.

3. Catalyst of clay minerals for the hydratisation of olefins, produced according to claims 1 and 2.

## Revendications

1. Procédé de fabrication d'un catalyseur à partir de minéraux argileux pour l'hydratation des oléfines ayant 2 à 3 atomes de carbone en les alcools correspondants, à partir d'acide phosphorique et d'un matériau de support, caractérisé en ce que tout d'abord, dans un premier stade, on traite avec un acide une argile renfermant essentiellement de la montmorillonite ou d'autres minéraux du groupe de la montmorillonite ne contenant pas de potassium tout en ayant le réseau cristallin de la montmorillonite, qui n'est pas contaminée par plus de 3% en poids de minéraux d'accompagnement comme le quartz, le feldspath et le mica et qui peut contenir jusqu'à 0,5% en poids de $K_2O$, jusqu'à ce qu'elle présente, éventuellement par une addition d'alumine précipitée, une teneur en $Al_2O_3$ de 13-18% en poids et une surface de 200-400 m²/g, de préférence de 240-300 m²/g, après quoi on ajoute 5 à 15% en poids, par rapport à la substance sèche totale d'un ou plusieurs oxydes de métaux du VIe sous-groupe du système périodique, puis on moule par compression à une teneur totale en eau de 20 à 35% en poids, on calcine à 500-800° C et l'on traite ensuite le matériau de support moulé dans un second stade avec de l'acide jusqu'à ce qu'il présente une teneur en $Al_2O_3$ de 1 à 5% en poids, de préférence de 1 à 3% en poids, et une surface de 150 à 250 m²/g, de préférence de 180 à 220 m²/g, et en ce qu'on imprègne le support ainsi obtenu de manière connue avec de l'acide phosphorique.

2. Procédé selon la revendication 1, caractérisé en ce que partant d'une terre décolorante hautement active déjà traitée une fois à l'acide, préparée à partir d'une argile à haute teneur en montmorillonite qui contient moins de 0,1% en poids de $K_2O$, dont le rapport pondéral $(Al_2O_3+Fe_2O_3):SiO_2$ s'élève à 1:3,5 à 1:4,5, dont la teneur en $Al_2O_3$ est éventuellement réglée par addition d'alumine précipitée à 16-18% en poids et dont la surface spécifique s'élève à 200-400 m²/g, de préférence à 240-300 m²/g, on ajoute à cette terre décolorante 5 à 15% en poids par rapport à la substance sèche totale, un ou plusieurs oxydes du VIe sous-groupe du système périodique, on la moule par compression en présence d'une teneur totale en eau de 20 à 35% en poids et on la soumet après calcination entre 500 et 800°C à un traitement acide, la teneur en oxyde d'aluminium étant abaissée à 1-5% en poids, de préférence à 1-3% en poids, et la surface spécifique à 150-250 m²/g, de préférence à 180-220 m²/g, après quoi on imprègne le support de contact ainsi obtenu de manière connue avec de l'acide phosphorique.

3. Catalyseur à partir de minéraux d'argile pour l'hydratation des oléfines, préparé selon les revendications 1 et 2.